# EUROPEAN PATENT SPECIFICATION

(11) **EP 1 067 137 B1**
(45) Date of publication and mention of the grant of the patent: **15.07.2009**
(21) Application number: 00119187.3
(22) Date of filing: 22.05.1992
(51) Int. Cl.: C07K 5/06

(54) **Granules of alpha-L-aspartyl-L-phenylalnine methyl ester**
Granulat des Alpha-Aspartyl-Phenylalanin-Methylesters
Granulés de l'ester-méthylique de l'alpha-aspartyl phénylalanine

(30) Priority: 24.05.1991 JP 22252591
(43) Date of publication of application: 10.01.2001
(62) Divisional of application: 92108705.2
(73) Proprietor: Ajinomoto Co., Inc., Tokyo 104 (JP)
(72) Inventor: Kishimoto, Shinichi c/o Ajinomoto Co.,Inc., Kawasaki-shi, Kanagawa-ken (JP); Yasaki, Akihiko c/o Ajinomoto Co., Inc., Kawasaki-shi, Kanagawa-ken (JP)
(74) Representative: Strehl Schübel-Hopf & Partner

(56) References cited:
- EP-A- 0 362 706
- DATABASE WPI Derwent Publications Ltd., London, GB; AN 1990-250664 XP002162622 Y SHIOKAWA ET AL.: "Production of granulated dipeptide sweetening comprises spray drying slurry containing crystals of alpha-l-aspartyl-l-phenylalanine lower alkyl ester and granulating" & JP 02 174650 A (TOSOH CORPORATION), 7 June 1990 (1990-06-07)

## Description

The present invention relates to granules of α-L-aspartyl-L-phenylalanine methyl ester (hereinafter referred to as "α-APM") having improved water-solubility.

α-APM is a dipeptide sweetener having a sweetness of about 200 times that of sucrose (cane sugar). Because of the extremely good sweetness and low calories, it has become used significantly as a diet sweetener in these days, and the worldwide demand for it is presumed to be over 10,000 tons before 1995.

Since α-APM is a bracing sweetener material which has little bitterness and no bad aftertaste, though in general high-sweetness sweeteners often have the same, it has widely been used and popularized as a low-calory sweetener. However, the drawback of the physical property of a poor dispersibility and solubility in water has often been pointed out. Therefore, in order to obtain α-APM having excellent solubility, various Investigations for granulating It or foaming and tableting it have heretofore been made by adding a vehicle or disintegrator thereto.

Chem. Abstr. 113 (1990), Abstract no. 130927v teaches that the flowability of APM may be improved by granulation and discloses granular APM having an average grain size of 74.5 µm. There is no indication, however, of the upper or lower limit of the grain size of the granules.

EP-A-0 255 092 discloses a method for producing granules of α-APM having an improved solubility. The granules have a preferred size of 2000 to 4000 µm.

DE-2 415 557 C2 discloses a method for improving the solubility of dipeptide sweeteners, such as APM, by mixing APM with an organic acid, such as citric acid, maleic acid or adipic acid.

However, incorporation of a vehicle into α-APM would often be problematic in particular uses. Therefore, high-purity and high-solubility α-APM is strongly desired.

As an attempt to improve the solubility of α-APM while maintaining the high purity thereof as it is, there are known a method of spray-drying a slurry of α-APM (Japanese patent Publication 58-20558); and a method of granulating α-APM to which water has been added to a specified water content (Japanese Patent Application Laid-Open No. 59-95862).

On the other hand, α-APM is known to be obtained in various forms of crystals in the process of its production. Among these, IB crystals have a higher solubility than IIA crystals and IIB crystals as dry crystals.

According to EP 0 119 837 B1 APM crystals having excellent storage stability are obtained by transforming the originally present APM I-type crystals into II-type crystals by drying them at a temperature in the range of 80°C to less than 150°C and molding and granulating the obtained II-type crystals.

Even though this document mentions a granulation step, it does not disclose any further step of selecting a certain range of the granule size of the obtained product.

However, IB form crystals of α-APM are known to still contain crystals having a low solubility. As an example, there is mentioned the fact that the crystal habit of either bundle-like crystal form or needle-like crystal form causes noticeable difference in the dispersibility and solubility of crystals in water. (The former bundle-like crystals have higher dispersibility and solubility in water than the latter.)

Of such IB needle-like crystals, some would often need a long time for dispersing or dissolving in water, which is almost comparable to IIA crystals or IIB crystals, depending upon the crystallization and drying conditions employed In preparing them.

Needle-like crystals as referred to herein designate those which are obtained by cooling crystallization under ordinary stirring condition without forming a pseudo solid phase.

On the other hand, bundle-like crystals as referred to herein with respect to the crystal habit designate those which are obtained by cooling crystallization of an α-APM solution under no stirring condition via its pseudo solid phase, as described in Japanese Patent Publication No. 2-45638 (crystallization of α-APM). When the crystals are observed with a scanning type electronic microscope under magnification, they are found to be crystal aggregates where plural needle-like crystals are bundled to seemingly form one crystal.

The object of the present invention is to improve the solubility of crystals of α-APM containing IB crystals having a poor solubility to such a degree that the time necessary for dissolving them is at least half or less of the time necessary for dissolving the original crystal powder of the compound.

The present inventors made earnest and repeated investigations for the purpose of attaining the object and, as a result, have found that the solubility of crystals of α-APM containing IB crystals may be improved by granulating the crystals to grains having a particular grain size. On the basis of the finding. they have achieved the present invention.

A binder is used for granulating and shaping α-APM, and one or more suitably selected from water and aqueous solutions of alcohols, saccharides and Inorganic materials may be used as the binder.

Granulation of α-APM may be effected by any method of mixing granulation, powder compression granulation, extrusion granulation, fluidizing granulation, rolling granulation, pulverizing granulation and others. From the viewpoint of the low heat load and for the purpose of evading any complicated process, dry granulation such as powder compression granulation is industrially advantageous.

The grain size of the granulated product containing IB crystals in an amount of 90 wt.% or more must be within the range of from 100 to 500 µm, preferably from 150 to 300 µm.

This is because if the grain size is less than 100 µm, the water-dispersibility of the granules would be poor so that dissolution of the granules would need much time. On the contrary, if it is more than 500 µm, the contact area between water and grains would unfavorably decrease so that improvement of the water-solubility of the grains could not be expected and the time necessary for dissolving them in water could not be reduced to half or less of that necessary for dissolving the original powder of the compound therein.

Therefore, the size of the granulated product is to be from 100 to 500 µm. Where the size of the granulated product satisfies the defined range, the time necessary for dissolving the granulated low-solubility IB crystals of α-APM in water may be reduced to about half or less of the time necessary for dissolving the original powder of the compound therein.

On the other hand, where the content of IB crystals is low in α-APM to be granulated, the solubility of the original powder of the compound of itself is low. Therefore, the effect of improving the solubility of the granulated product from it could be expected even in a broad range for the grain size. That is, the grain size of the granulated product may well be within the range of from 100 to 1400 µm, preferably from 150 to 500 µm. Satisfying the defined range, the time necessary for dissolving the granulated product in water could be reduced to about half or less of the time necessary for dissolving the original crystal powder of the compound therein, like a high-purity product.

In accordance with the present invention, even a low-solubility powdery α-APM of IB crystals may be formed into granules having an Improved a high solubility in water.

Therefore, where the granules of α-APM of the present Invention are added to drinks or others, the time necessary for dissolving them may be reduced and the distribution of the operation time for dissolution may also be reduced. As a result, the granules of α-APM of the present invention may be handled with high efficiency.

### Example:

A powder of α-APM was prepared in accordance with the method mentioned below to be a sample for evaluation.

An aqueous α-APM solution was crystallized by ordinary cooling crystallization, whereupon the solution was stirred (stirring crystallization not forming a pseudo solid phase). By centrifugation, wet α-APM crystals having a water content of 60 wt.% were taken out, and they were dried in a laboratory small-sized fluidizing drier at 90 ° C for 30 minutes. Then, they were milled in a laboratory small-sized centrifugal mill (5000 rpm, using 1 mm φ screen) to obtain an original IB needle-like crystal powder having a size of less than 50-60 µ in average.

### This is sample A.

380 ml of an aqueous raw material solution containing 17.7 kg of α-APM as dissolved therein (55 ° C, initial concentration of α-APM 4.4 wt.%) was previously put in a cooling jacket-combined stainless steel crystallizer having a diameter of 400 mm and having a cooling plate in the inside thereof, and a coolant of a temperature of 0 ° C was circulated into the cooling jacket and the cooling plate whereby the content in the crystallizer was cooled for 3 hours.

After about one hour, the whole solution became a pseudo solid phase, and the pseudo solid phase α-APM crystals were dropped into a receiver as equipped with a cooling coil and a stirrer and pulverized therein to form a slurry, which was further cooled. (The cooling was effected from 16 ° C to 7 ° C in the receiver.)

The slurry thus obtained was filtered and dewatered In a centrifugal separator having a diameter of about 91 cm to obtain wet α-APM crystals having a water content of 30 wt.%.

The wet α-APM crystals thus obtained by the pseudo solid phase method were dried In a laboratory small-sized fluidizing drier at 90 ° C for 30 minutes and then milled in a a laboratory small-sized centrifugal mill (5000 rpm, using 1 mm φ screen) to obtain an original IB bundle-like crystal powder.

### This is sample B.

An aqueous α-APM solution was crystallized by ordinary cooling crystallization, whereupon the solution was stirred (stirring crystallization not forming a pseudo solid phase). By centrifugation, wet α-APM crystals having a water content of 60 wt.% were taken out, and they were continuously fed into a Micron Drier (manufactured by Hosokawa Micron Co.) via a screw feeder and air-dried therein with hot air of a temperature of 140 ° C to obtain an original IIA needle-like crystal powder.

40 wt.% of the thus obtained original IIA needle-like crystal powder and 60 wt.% of the previously obtained original IB needle-like crystal powder (sample A) were blended to give sample C.

Each of the samples A, B and C were shaped by compression shaping to obtain compressed flakes, which were then pulverized in a fine granulator and sieved through JIS standard sieves to obtain α-APM granules of several groups each having the grain size range as shown In Table 1 below.

The dry compression shaping followed by pulverizing was conducted by the use of Roller Compactor Model WP90 × 30 (manufactured by Turbo Industrial Co.), and the feed amount of the original powder In the compression shaping step was 40 g/min, the roll pressure was 50 kg/cm².G and the roll rotating number was 12 rpm. The screen of the fine granulator as used in the pulverizing step was a 12-mesh screen (with opening of 1400 µm).

The dissolution time of the α-APM granules thus obtained was measured by the method mentioned below, and the results obtained are shown in Table 1.

First, 2 liters of water were previously put in a 3-liter beaker and stirred with a magnetic stirrer.

The size of the stirrer to be used for the stirring was 70 mm x 15 mm φ and the rotation number was set to be 350 rpm with Whatman Dataplate 440. The temperature of the water was kept to be 20 ° C by the use of a hot plate. 8 g of a sample was put into the water under the determined condition, whereupon the time needed for completely dissolving it in the water was measured to be the dissolution time of the sample.

**TABLE 1**

| Grain Size (µm) | Dissolution Time (min) | | |
|---|---|---|---|
| | A | B | C |
| 850 to 1400 | 38 | 35 | 38 |
| 500 to 850 | 25 | 24 | 27 |
| 300 to 500 | 18 | 17 | 19 |
| 180 to 300 | 11 | 9 | 12 |
| 150 to 180 | 8 | 7 | 20 |
| 100 to 150 | 15 | 10 | 36 |
| (Original Powder before Granulation) | 30 | 15 | 60 |

## Claims

1. Granules of α-APM containing IB crystals of α-APM and having a grain size within the range of from 100 to 1400 µm which are dissolvable in water within a period of half or less of the time necessary for dissolving the original crystal powder of the compound.

2. The granules of claim 1 which are obtainable by granulating a composition containing IB crystals of α-APM and sieving the obtained granules.

3. The granules of claim 1 or 2 having a grain size within the range of from 150 to 500 µm.

## Patentansprüche

1. Granulate von α-APM, die IB-Kristalle von α-APM enthalten und eine Körnchengröße im Bereich von 100 bis 1400 µm aufweisen, die in Wasser innerhalb der Hälfte oder weniger des Zeitraums, der für das Auflösen des ursprünglichen Kristallpulvers der Verbindung erforderlich ist, löslich sind.

2. Granulate nach Anspruch 1, die durch Granulieren einer Zusammensetzung, die IB-Kristalle von α-APM enthält, und Sieben der erhaltenen Granulate erhältlich sind.

3. Granulate nach Anspruch 1 oder 2 mit einer Körnchengröße im Bereich von 150 bis 500 µm.

## Revendications

1. Granulés d'α-APM contenant des cristaux IB d'α-APM et ayant une taille de grains dans l'intervalle de 100 à 1400 µm qui peuvent être dissous dans de l'eau sur une durée moitié ou inférieure au temps nécessaire pour dissoudre la poudre de cristaux d'origine du composé.

2. Granulés selon la revendication 1 qui peuvent être obtenus par granulation d'une composition contenant des cristaux IB d'α-APM et par tamisage des granulés obtenus.

3. Granulés selon la revendication 1 ou 2 ayant une taille de grains dans l'intervalle de 150 à 500 µm.
